(19) Europäisches Patentamt
European Patent Office
Office européen des brevets

(11) Publication number: **0 002 320**
**B1**

(12) # EUROPEAN PATENT SPECIFICATION

(45) Date of publication of patent specification: **29.04.81**

(21) Application number: **78300582.0**

(22) Date of filing: **02.11.78**

(51) Int. Cl.³: **C 07 D 498/04**
**//(C07D498/04, 263/00, 205/00)**

(54) **Process for the preparation of clavulanic acid derivatives and compounds used as intermediates therein.**

(30) Priority: **30.11.77 GB 4998877**
**07.01.78 GB 60378**

(43) Date of publication of application:
**13.06.79 Bulletin 79/12**

(45) Publication of the grant of the European patent:
**29.04.81 Bulletin 81/17**

(84) Designated Contracting States:
**CH DE FR GB**

(56) References cited:
**DE - A - 2 646 000**
**DE - A - 2 728 533**

(73) Proprietor: **BEECHAM GROUP LIMITED**
**Beecham House Great West Road**
**Brentford, Middlesex (GB)**

(72) Inventor: **Stirling, Irene**
**80 Woodcrest Walk**
**Reigate Surrey (GB)**
Inventor: **Harbridge, John Barry**
**109 St.Andrews Road**
**Coulsdon Surrey (GB)**

(74) Representative: **Cole, William Gwyn, Dr. et al,**
**Beecham Pharmaceuticals Research Centre Yew Tree Bottom Road**
**Epsom, Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 002 320 B1

# Process for the preparation of clavulanic acid derivatives and compounds used as intermediates therein

The present invention relates to a process for the preparation of esters of clavudiene, to intermediates useful in that process and to the preparation of said intermediates.

A use for the compounds of the formula (I):

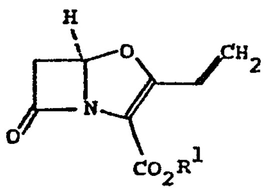

(I)

wherein $R^1$ is a group such that $CO_2R^1$ is an ester group was disclosed in Belgian Patent No. 847044. Such compounds have been previously prepared from acyl derivatives of esters of clavulanic acid. It has now been discovered that the compounds of the formula (I) are readily prepared in good yields from N-acylcarbamoylclavulanic acid esters. N-substituted carbamoylclavulanic acid esters are disclosed in DE—OS 2 646 000.

Accordingly the present invention provides a process for the preparation of a compound of the formula (I) which process comprises the base catalysed elimination of carbon dioxide and a compound of the formula (II):

$$H_2N.COR^2 \qquad (II)$$

wherein $R^2$ is an inert moiety, from a compound of the formula (III):

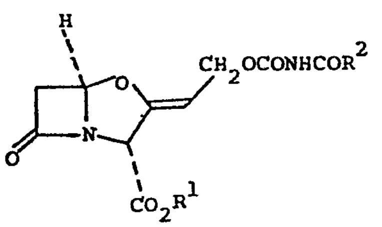

(III)

wherein $R^1$ is as defined in relation to formula (I) and $R^2$ is as defined as in relation to formula (II).

Suitably $R^2$ is an alkyl, alkenyl, alkynyl, aryl, alkoxy, aryloxy, aralkoxyl or aralkyl group which may be inertly substituted if desired.

Such groups $R^2$ which do not contain aryl moieties generally contain from 1 to 8 carbon atoms and more usually from 1 to 4 carbon atoms. Such groups $R^2$ which contain aryl moieties may contain from 1 to 4 carbon atoms other than those of the aryl moiety. Most suitably the aryl moiety in such cases is a phenyl or substituted phenyl group.

Thus suitable groups $R^2$ include methyl, ethyl, ethoxy, propyl, butyl, trichloromethyl, trifluoromethyl, chloromethyl, dichloromethyl, methoxymethyl, methoxyethyl, allyl, cyclohexyl, phenoxy, phenyl, chlorophenyl, bromophenyl, nitrophenyl, methoxyphenyl, phenylethyl, benzyl, benzyloxy, nitrobenzyl, chlorobenzyl and bromobenzyl groups.

A preferred group $R^2$ is the phenyl group (owing to the ready availability of the intermediate compound of the formula $C_6H_5CONCO$).

Suitable hydrolysable groups $R^1$ include alkyl groups of up to 4 carbon atoms optionally substituted by halogen, alkoxyl of up to 4 carbon atoms or acyloxyl of up to 4 carbon atoms. One favoured group $R^1$ is the methyl group. Another favoured group $R^1$ is the methoxymethyl group.

Suitable hydrogenolysable groups $R^1$ include optionally substituted benzyl esters such as benzyl, bromobenzyl, chlorobenzyl, methoxybenzyl, nitrobenzyl or the benzhydryl ester. One favoured group $R^1$ is the benzyl group. Another favoured group $R^1$ is the p-bromobenzyl group. A further favoured group $R^1$ is the p-nitrobenzyl group. Yet another favoured group $R^1$ is the p-methoxybenzyl group.

The base used to initiate the elimination from the compound of the formula (III) may be a tertiary amine such as a triloweralkylamine such as trimethylamine or triethylamine.

Other suitable bases include a base which has a pKa in excess of about 9.5 and which will not cause rapid degradation of the $\beta$-lactam ring such as N-methylpyrrolidone, N-methylpiperidine, N-methylmorpholine, 1,5-diazabicyclo[5,4,0]undec-5-ene, and 1,5-diazabicyclo[4,3,0]-non-5-ene.

The base need only be present in catalytic quantities, for example from 5 to 20 mole %, preferably 10—15 mol %.

The reaction is normally carried out in an inert organic solvent such as dichloromethane, chloroform, dimethylformamide, tetrahydrofuran, acetonitrile, ethyl acetate or acetone.

In general the reaction may be carried out at any convenient non-extreme temperature, for example from 0° to 30°C. It is preferred to carry out the reaction at about 0°C.

When the reaction is substantially complete (for example as judged by the tlc using permanganate spray for identification) the diene may be used *in-situ* or obtained in isolated form. In order to isolate the diene the solution may optionally be washed with water (if immiscible), dried and evaporated. The diene may then be purified by chromatography, for example over silica gel using an elution solvent such as ethyl acetate/cyclohexane mixtures.

The present invention also provides the compounds of the formula (III) as hereinbefore defined.

In yet a further aspect this invention provides a process for the preparation of a compound of the formula (III) which process comprises the reaction of a compound of the formula (IV):

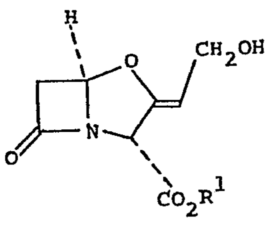

(IV)

wherein $R^1$ is as defined in relation to formula (I) with a compound of the formula (V):

$$O{=}C{=}N{-}CO{-}R^2 \qquad (V)$$

wherein $R^2$ is as defined in relation to formula (III).

The addition reaction may take place in a solvent and at a temperature as described for the previously described elimination reaction. Under such conditions the reaction is over quickly, for example often in under 10 minutes.

The desired compound of the formula (II) may be isolated by evaporating the solvent and the remaining reagents and/or by chromatography.

In yet another aspect this invention provides the compound of the formula (I) wherein $R^1$ is a p-nitrobenzyl group. p-Nitrobenzyl clavudiene has the advantage of being a crystalline solid of good stability so that it is capable of serving as a particularly suitable intermediate in view of its storage properties.

It has been observed that trituration of the crude p-nitrobenzyl clavudiene under dry diethyl ether can aid the solidification and crystallisation processes. This may be due to the solubility of side products (such as trichloroacetamide) in the ether.

Suitable compounds of the formula (IV) include those described and especially those individually claimed in British Patent No. 1508978.

Example 1

Benzyl 9-(N-trichloroacetyl)carbamoylclavulanate

To a solution of benzylclavulanate (6.3g) in tetrahydrofuran (dry, redistilled, 100 ml) was added a solution in $CH_2Cl_2$ of trichloroacetyl isocyanate (1.0M, 22.8 ml, slight excess). After 2 minutes at 5°C tlc (ethylacetate-cylcohexane 1:1—$SiO_2$) showed the absence of starting material. The compound was isolated by evaporation of the solvent; 10.7 g quantitative.

Ir (1/f) 3600 (broad) 1810, 1750 and 1700 (sh). Nmr ($CDCl_3$): $\delta$ 3.04(1H, d, *J* 17Hz, (6-$\beta$-C*H*) 3.48(1H, dd, *J* 17 and 3Hz, 6-$\alpha$-C*H*), 4.84(3H, s, 9-$CH_2$ and 8-C*H*) 5.06 (1H, s, 3-C*H*), 5.15(2H, s, $CH_2C_6H_5$) 5.67(1H, d, *J* 3Hz, 5-C*H*) 7.28(5H, s, $CH_2CH_2C_6H_5$), 8.49(1H, s, N*H*, exchanges with $D_2O$).

Example 2

Benzyl 9-(N-phenylacetyl)carbamoylclavulanate

To a stirred solution of benzyl clavulanate (1.9 g) in tetrahydrofuran (15 ml) was added crude freshly distilled phenylacetyl isocyanate (1.3 g) dropwise from a dropping pipette. Heat was evolved. After cooling to room temperature the tetrahydrofuran was evaporated under reduced pressure and unreacted benzyl clavulanate (which was more polar than the product) separated by column chromatography on silica gel using ethyl acetate and cyclohexane (1:1) as eluents. The product was an almost colourless oil (2.5 g). It had nmr. $\delta$($CDCl_3$) 2.95(1H, d, J 17Hz, 6$\beta$-C*H*), 3.41(1H, dd, *J* 17 and 2.5Hz, 6$\alpha$-C*H*), 4.0(2H, s, $COCH_2Ph$), 4.6—5.0(3H, m, 9-$CH_2$ and 8-C*H*) 5.15(1H, s, 3-C*H*) 5.17(2H, s, $OCH_2Ph$), 5.77 (1H, d, *J* 2.5Hz, 5-C*H*) 7.27 and 7.23(10H, 2s, $2xC_6H_5$) and 8.23(1H, s, N*H*).

Example 3

Benzyl clavudiene

To a solution of the N-acylcarbamoyl compound of Example 1 (0.01 mole) in dichloromethane (or in dimethylformamide, tetrahydrofuran or acetonitrile) (50 ml) was added triethylamine (or trimethylamine) in tetrahydrofuran (about 5 mole%) at ambient temperature. The reaction was followed by tlc and when the starting material had disappeared (10 mins) the diene was isolated by column chromatography on silica gel using ethylacetate and cyclohexane (1:1) as eluent.

Evaporation of the fractions containing the benzyl clavudiene yielded the desired product as an oil (0.009 mole).

(If desired the initially produced solution of the diene may be employed for further reaction without isolation.)

Example 4

p-Nitrobenzyl clavudiene

p-Nitrobenzylclavulanate (3.34 g) in methylene chloride (50 ml) was treated with a solution of trichloroacetyl isocyanate in methylene chloride (10 ml) of 1 molar solution) at 0°C. The mixture was stirred at 0° until the starting material disappeared (as judged by tlc). A solution of trimethylamine in tetrahydrofuran (2.2 ml of 1.8 molar solution) was then added and the temperature allowed to rise to room temperature and the mixture then stirred for a further 2 hours. At this time, tlc showed that the intermediate trichloroacetylcarbamoyl clavulanate had been converted to the diene (the diene is only faintly blue fluorecent under 366 nm, uv on tlc). The solution was concentrated under reduced pressure to small volume (about 5—10 ml) and triturated with sodium-dried ether. The resulting off-white solid was filtered off, washed with ether and dried in a desicator to yield the title compound (2.4 g). Ir. (Nujol): 1805, 1705, 1630, 1605 $cm^{-1}$.

Example 5

Methoxymethyl Clavudiene

Methoxymethyl clavulanate (4.86 g) in methylene chloride (50 ml) was treated with a

solution of trichloroacetyl isocyanate in methylene chloride (20 ml of 1M solution; 1 equivalent) at 0°. The reaction mixture was stirred in an ice-bath until the disappearance of starting material (as judged by t.l.c.; ~1.5 hours).

The intermediate acylcarbamate was treated with a solution of trimethylamine in tetrahydrofuran (4.4 ml of 1.8M solution) and t.l.c. showed complete conversion to the diene after stirring between 0°—4° for 1 hour. The solution was washed with 20% acetic acid solution, water, saturated sodium bicarbonate, water, brine and evaporated to an oil. The crude product was passed quickly down a column of silica-gel, eluting with ethyl acetate/cyclohexane 1:1 and was obtained in a yield of 80% as a colourless oil on evaporation of the eluate.

$[\alpha]_D^{20} = +14°$ (C = 4.2; $CHCl_3$). $\nu_{max}$ ($CHCl_3$) 1820, 1715, 1635, 1570cm$^{-1}$; $\delta$($CCl_4$) 3.42(1H, d, J 17.5Hz, 6$\beta$-C*H*), 3.43(3H, s, $CH_2OCH_3$) 3.79(1H, dd, J 17.5 and 3.5Hz, 6$\alpha$-C*H*), 5.17, 5.37(2H, ABq, J 6Hz, $CH_2OCH_3$), 5.57(1H, dd, J 11 and 2Hz, CH=CHH), 5.83(1H, dd, J 17 and 2Hz, CH=CHH), 5.88 (1H, dd, J 3.5 and 1.5Hz, 5-C*H*), 7.03(1H, dd, J 17 and 11 Hz, C*H*=$CH_2$).

Example 6

Methoxymethyl N-benzoylcarbamoylclavulanate

To a stirred solution of methoxymethyl clavulanate (4.8 g) in dichloromethane (25 ml) was added excess benzoylisocyanate (about 5 g). The mixture was evaporated to dryness under reduced pressure, the residue dissolved, as far as possible, in ethyl acetate (20 ml), and the suspension subjected to column chromatography on silica gel using ethyl acetate — cyclohexane (1:1) as elution solvent. Fractions containing the desired product (by t.l.c.) were cooled and the deposited benzamide filtered off and discarded. The filtrate was evaporated to dryness under reduced pressure, and treated with dichloromethane (10 ml). Benzamide was again removed by filtration, and the filtrate evaporated to dryness in vacuo, to leave a pale yellow oil (5.1 g).

This I.r. (film): 3340 (broad), 1805, 1760, 1695 cm$^{-1}$; $\delta$ ($CDCl_3$): 3.09 (1H, d, *J* 17Hz, 6-—C*H*), 3.50 (3H, s, $OCH_3$), 3.55 (1H, dd, *J* 17 and 3Hz, 6-—C*H*), 4.70—5.05 (3H, m, 8-C*H* and 9-$CH_2$), 5.10(1H, s, 3-C*H*), 5.36, 5.40 (2H, ABq, *J* 6 Hz, $OCH_2O$), 5.77(1H, d, *J* 3Hz, 5-C*H*), 7.30—8.1(5H, m, $C_6H_5$) and 8.55(1H, br.s., N*H*, (exchanges with $D_2O$).

Example 7

Methoxymethyl ester of clavudiene

To a solution of the acylcarbamate produced in Example 6 (0.1 g) in dry tetrahydrofuran (1 ml), cooled to 2—3°C, was added triethylamine (0.01 ml). The mixture was stirred for 40 mins, by which time t.l.c. showed that the starting material ahd almost completely reacted, and a faster-running zone, blue-fluorescent at 360 nm, had been formed. Evaporation of the solvent under reduced pressure gave a mixture of oil and crystals. The mixture was extracted with $CCl_4$ (3 ml), filtered and the filtrate evaporated to give the diene as an oil (0.06 g). I.r. 1808 and 1715 cm$^{-1}$ (film).

Example 8

Benzyl N - benzoylcarbamoylclavulanate

To a stirred solution of benzylclavulanate (2.89 g) in dry dichloromethane (20 ml) was added benzoyl isocyanate (2.5 g). The mixture was evaporated to dryness under reduced pressure, and the residue dissolved (as far as possible) in ethyl acetate — cyclohexane 1:1 (10 ml). The solution was filtered and the filtrate subjected to column chromatography on silica gel using the above solvent mixture. Fractions containing the desired product (by t.l.c.) were cooled, and the deposited benzamide filtered off and discarded. The filtrate was evaporated to dryness under reduced pressure and redissolved in dichloromethane (the minimum volume, about 3—4 ml). Small amounts of benzamide were again filtered off, and the filtrate added to isopropanol (40 ml). On scratching and cooling, the desired product crystallised slowly (during 48hr. at 2—3°C), and it was collected by filtration, washed quickly with cold ether and dried in vacuo to yield 1.1 g, m.p. 80°C (Kofler hot bench, rapid decomp.).

I.r. (Nujol mull): 3260, 1805, 1760, 1750 and 1695cm$^{-1}$; $\delta$($CDCl_3$): 3.04(1H, d, *J* 17Hz, 6$\beta$-C*H*), 3.53(1H, dd, *J* 17 and 3Hz, 6$\alpha$-C*H*), 4.7—5.0(3H, m, 9-$CH_2$ and 8-C*H*), 5.10(1H, s, 3-C*H*), 5.21(2H, s, Ph$CH_2$), 5.73(1H, d, *J* 3 Hz, 5-C*H*), 7.3—8.0(10H, m, 2 x $C_6H_5$) and 8.28(1H, bs, exchanges with $D_2O$, N*H*).

Example 9

Benzyl ester of clavudiene

To a solution of the N-benzoylcarbamate produced in Example 8 (0.1 g) in tetrahydrofuran (2 ml) was added triethylamine (0.01 ml). On standing at 2—3°C t.l.c. showed a faster moving zone which fluoresced strongly at 360 nm. After 40 min. the reaction was virtually complete.

**Claims**

1. A process for the preparation of a compound of the formula (I):

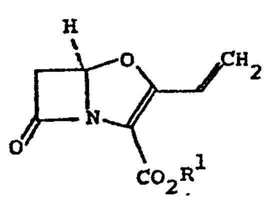

(I)

wherein $R^1$ is a group such that $CO_2R^1$ is an ester group; which process comprises the base catalysed elimination of carbon dioxide and a

compound of the formula (II):

$$H_2N.COR^2 \qquad (II)$$

wherein $R^2$ is an inert moiety, from a compound of the formula (III):

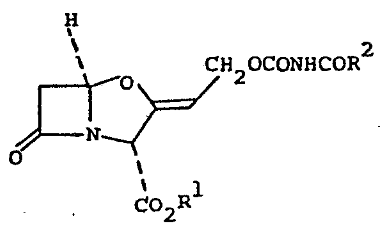

(III)

wherein $R^1$ is as defined in relation to formula (I) and $R^2$ is as defined as in relation to formula (II).

2. A process as claimed in claim 1 wherein $R^2$ is a methyl, ethyl, ethoxy, propyl, butyl, trichloromethyl, trifluoromethyl, chloromethyl, dichloromethyl, methoxymethyl, methoxyethyl, allyl, cyclohexyl, phenyl, phenoxy, chlorophenyl, bromophenyl, nitrophenyl, methoxyphenyl, phenylethyl, benzyl, benzyloxy, nitrobenzyl, chlorobenzyl or bromobenzyl group.

3. A process as claimed in claim 1 wherein $R^2$ is a phenyl group.

4. A process as claimed in any of claims 1—3 wherein $R^1$ is an alkyl group of up to 4 carbon atoms optionally substituted by halogen, alkoxyl of up to 4 carbon atoms or acyloxyl of up to 4 carbon atoms.

5. A process as claimed in claim 4 wherein $R_1$ is a methyl or methoxymethyl group.

6. A process as claimed in any of claims 1—3 wherein $R^1$ is a benzyl or substituted benzyl group.

7. A process as claimed in claim 6 wherein $R^1$ is a benzyl, p-bromobenzyl, p-nitrobenzyl or p-methoxybenzyl group.

8. A process as claimed in any of claims 1—7 wherein the base employed is a tertiary amine.

9. A process as claimed in claim 8 wherein the amine is trimethylamine or triethylamine.

10. A process as claimed in any of claims 1—9 carried out at 0°C.

11. A compound of the formula (III) as shown in claim 1 wherein $R^1$ and $R^2$ are as defined in any of claims 1—7.

12. A process for the preparation of a compound as claimed in claim 11 which process comprises the reaction of a compound of the formula (IV):

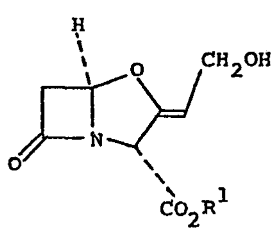

(IV)

wherein $R^1$ is as defined in any of claims 1,4,5,6 and 7 with a compound of the formula (V):

$$O{=}C{=}N{-}CO{-}R^2 \qquad (V)$$

wherein $R^2$ is as defined in relation to any of claims 1—3.

**Revendications**

1. Procédé de préparation d'un composé de formule (I):

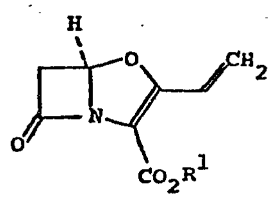

(I)

où $R^1$ est un groupe tel que $CO_2R^1$ représente un groupe ester; procédé caractérisé en ce qu'il comprend l'élimination catalysée par une base d'anhydride carbonique et d'un composé de formule (II):

$$H_2N.COR^2 \qquad (II)$$

où $R^2$ est un groupe inerte, à partir d'un composé de formule (III):

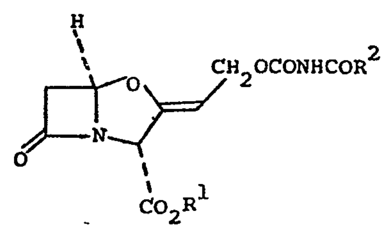

(III)

où $R^1$ a la signification indiquée en ce qui concerne la formule (I) et $R^{23}$ a la signification indiquée en ce qui concerne la formule (II).

2. Procédé suivant la revendication 1, caractérisé en ce que $R^2$ est un groupe méthyle, éthyle, éthoxy, propyle, butyle, trichlorométhyle, trifluorométhyle, chlorométhyle, dichlorométhyle, méthoxyméthyle, méthoxyéthyle, allyl, cyclohexyle, phényle, phénoxy, chlorophényle, bromophényle, nitrophényle, méthoxyphényle, phényléthyle, benzyl, benzyloxy, nitrobenzyle, chlorobenzyle ou bromobenzyle.

3. Procédé suivant la revendication 1, caractérisé en ce que $R^2$ est un groupe phényle.

4. Procédé suivant l'une quelconque des revendications 1 à 3, caractérisé en ce que $R^1$ est un groupe alcoyle ayant jusqu'à 4 atomes de carbone, facultativement substitué par un halogène, un groupe alcoxy ayant jusqu'à 4 atomes de carbone ou acyloxy ayant jusqu'à 4 atomes de carbone.

5. Procédé suivant la revendication 4, caractérisé en ce que $R^1$ est un groupe méthyle ou méthoxyméthyle.

6. Procédé suivant l'une quelconque des

revendications 1 à 3, caractérisé en ce que $R^1$ est un groupe benzyle ou benzyle substitué.

7. Procédé suivant la revendication 6, caractérisé en ce que $R^1$ est un groupe benzyle, p-bromobenzyle, p-nitrobenzyle ou p-méthoxybenzyle.

8. Procédé suivant l'une quelconque des revendications 1 à 7, caractérisé en ce que la base utilisé est une amine tertiaire.

9. Procédé suivant la revendication 8, caractérisé en ce que l'amine est la triméthylamine ou la triéthylamine.

10. Procédé suivant l'une quelconque des revendications 1 à 9, caractérisé en ce qu'on opère à 0°C.

11. Composé de formule (III) comme indiquée dans la revendication 1, où $R^1$ et $R^2$ sont tels que définis dans l'une quelconque des revendications 1 à 7.

12. Procédé de préparation d'un composé suivant la revendication 11, caractérisé en ce qu'il comprend la réaction d'un composé de formule (IV):

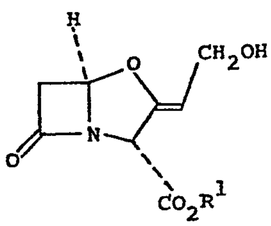

(IV)

où $R^1$ a la signification indiquée dans l'une quelconque des revendications 1, 4, 5, 6 et 7 avec un composé de formule (V):

$$O{=}C{=}N{-}CO{-}R^2 \qquad (V)$$

où $R^2$ a la signification indiquée dans l'une quelconque des revendications 1 à 3.

**Patentansprüche**

1. Verfahren zur Herstellung einer Verbindung der Formel (I)

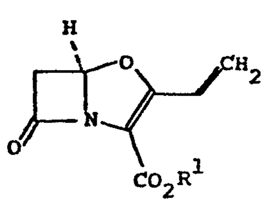

(I)

in der $R^1$ ein solcher Rest ist, daß der Rest $CO_2R^1$ eine Estergruppe ist, welches die durch eine Base katalysierte Abspaltung von Kohlendioxid und einer Verbindung der Formel (II)

$$H_2N.COR^2 \qquad (II)$$

in der $R^2$ ein inerter Rest ist, aus einer Verbindung der Formel (III) einschließt

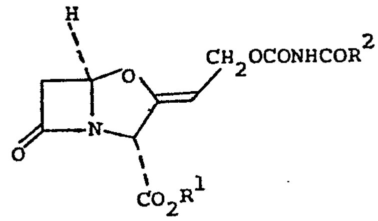

(III)

in der $R^1$ wie in bezug auf Formel (I) und $R^2$ wie in bezug auf Formel (II) definiert sind.

2. Verfahren gemäß Anspruch 1, wobei $R^2$ ein Methyl-, Äthyl-, Äthoxy-, Propyl-, Butyl-, Trichlormethyl-, Trifluormethyl-, Chlormethyl-, Dichlormethyl-, Methoxymethyl-, Methoxyäthyl-, Allyl-, Cyclohexyl-, Phenyl-, Phenoxy-, Chlorphenyl-, Bromphenyl-, Nitrophenyl-, Methoxyphenyl-, Phenäthyl-, Benzyl-, Benzyloxy-, Nitrobenzyl-, Chlorbenzyl- oder Brombenzyl-Rest ist.

3. Verfahren gemäß Anspruch 1, wobei $R^2$ ein Phenylrest ist.

4. Verfahren gemäß einem der Ansprüche 1 bis 3, wobei $R^1$ ein gegebenenfalls mit Halogenatomen substituierter Alkylrest mit bis zu 4 Kohlenstoffatomen, ein Alkoxyrest mit bis zu 4 Kohlenstoffatomen oder ein Acyloxyrest mit bis zu 4 Kohlenstoffatomen ist.

5. Verfahren gemäß Anspruch 4, wobei $R^1$ eine Methyl- oder Methoxymethylgruppe ist.

6. Verfahren gemäß einem der Ansprüche 1 bis 3, wobei $R^1$ ein Benzyl- oder substituierter Benzylrest ist.

7. Verfahren gemäß Anspruch 6, wobei $R^1$ ein Benzyl-, p-Brombenzyl-, p-Nitrobenzyl- oder p-Methoxybenzylrest ist.

8. Verfahren gemäß einem der Ansprüche 1 bis 7, wobei die verwendete Base ein tertiäres Amin ist.

9. Verfahren gemäß Anspruch 8, wobei das Amin Trimethylamin oder Triäthylamin ist.

10. Verfahren gemäß einem der Ansprüche 1 bis 9, durchgeführt bei 0°C.

11. Eine Verbindung der Formel (III) wie in Anspruch 1, in der $R^1$ und $R^2$ wie in den Ansprüchen 1 bis 7 definiert sind.

12. Verfahren zur Herstellung einer Verbindung gemäß Anspruch 11, welches die Umsetzung einer Verbindung der Formel IV

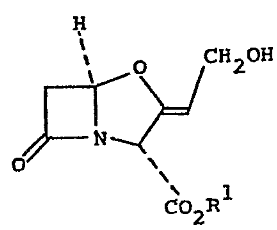

(IV)

in der $R^1$ wie in einem der Ansprüche 1, 4, 5, 6 und 7 definiert ist, mit einer Verbindung der Formel (V) einschließt.

$$O{=}C{=}N{-}CO{-}R^2 \qquad (V)$$

in der $R^2$ wie in einem der Ansprüche 1 bis 3 definiert ist.